# EUROPEAN PATENT APPLICATION

(11) **EP 4 338 748 A1**
(43) Date of publication of application: **20.03.2024**
(21) Application number: 22807465.4
(22) Date of filing: 10.05.2022
(51) Int. Cl.: A61K 38/10, A61P 25/28, C07K 7/06, C07K 7/08

(54) **AMYLOID-BETA AGGREGATION INHIBITOR, PHARMACEUTICAL COMPOSITION FOR AMYLOID-BETA AGGREGATION DISEASES, AND USE APPLICATION OF SAME**

(30) Priority: 11.05.2021 JP 2021080513
(71) Applicant: O-Force Co., Ltd., Kochi, 789-1931 (JP)
(72) Inventor: AKIZAWA Toshifumi, Hata-gun, Kochi 789-1931 (JP); SAITO Motoaki, Kochi-shi, Kochi 780-8520 (JP); NAKAMURA Rina, Hata-gun, Kochi 789-1931 (JP)
(74) Representative: Dehns
(86) International application number: PCT/JP2022/019786
(87) International publication number: WO 2022/239765

(57) **Abstract**

Provided is a novel drug that inhibits aggregation of amyloid-β, which causes Alzheimer's disease and the like. The present invention is directed to an amyloid-β aggregation inhibitor characterized by containing a peptide (A1) or (A2) below. (A1) A peptide having an amino acid sequence of 5 or more consecutive amino acid residues in the amino acid sequence of SEQ ID NO: 1: YKNMRETLVYLTHLDYDDTE (SEQ ID NO: 1). (A2) A peptide having an amino acid sequence obtained by performing deletion, substitution, or addition of 1 or 2 amino acids on the amino acid sequence of (A1) above.

## Description

### Technical Field

The present invention relates to an amyloid-β aggregation inhibitor, a pharmaceutical composition for an amyloid-β aggregation disease, and a use application of the same.

### Background Art

Alzheimer's disease, which predominantly occurs in old age, has become a serious issue due to the increasing proportion of the elderly in the population as a result of rising life expectancy. Alzheimer's disease is a progressive central neurodegenerative disease that results in cognitive dysfunction and memory loss. This disease is thought to be caused by fibrillar aggregates (amyloid fibrils) generated by aggregation of amyloid-β through intermolecular association in the brain. However, no clinically effective drugs have been implemented into practice, and further investigation of candidate drugs is required. This issue is not limited to Alzheimer's disease, but applies to all diseases caused by amyloid fibrils.

### Summary of Invention

### Technical Problem

It is an object of the present invention to provide a novel drug that inhibits aggregation of amyloid-β, which causes Alzheimer's disease and the like.

### Solution to Problem

The present invention is directed to an amyloid-β aggregation inhibitor characterized by containing a peptide (A1) or (A2) below.
(A1) A peptide having an amino acid sequence of 5 or more consecutive amino acid residues in the amino acid sequence of SEQ ID NO: 1
   YKNMRETLVYLTHLDYDDTE (SEQ ID NO: 1)
(A2) A peptide having an amino acid sequence obtained by performing deletion, substitution, or addition of 1 or 2 amino acids on the amino acid sequence of (A1) above

The present invention is directed to a pharmaceutical composition for an amyloid-β aggregation disease characterized by containing the peptide (A1) or (A2).

The present invention is directed to a method for inhibiting aggregation of amyloid-β, characterized by including adding the amyloid-β aggregation inhibitor of the present invention to a test subject.

The present invention is directed to a method for treating an amyloid-β aggregation disease, characterized by including administering the amyloid-β aggregation inhibitor of the present invention to a test subject.

### Advantageous Effects of Invention

The amyloid-β aggregation inhibitor of the present invention can inhibit the aggregation of amyloid-β caused by intermolecular association. This enables treatment, e.g., prevention, inhibition of progression, and amelioration, of amyloid aggregation diseases such as Alzheimer's disease caused by aggregation of amyloid-β.

### Brief Description of Drawings

[FIG. 1] FIG. 1 shows graphs showing the fluorescence intensities of reaction solutions in which peptides YS were added to Aβ25-35 before aggregation reaction.
[FIG. 2] FIG. 2 shows graphs showing the inhibition ability IC50 of peptides YS against aggregation of Aβ25-35.
[FIG. 3] FIG. 3 shows electron micrographs showing the degrees of aggregation in reaction solutions in which peptides YS were added to Aβ25-35 before aggregation reaction.
[FIG. 4] FIG. 4 is a graph showing the fluorescence intensities of reaction solutions in which peptides YS were added to an Aβ25-35 aggregate.
[FIG. 5] FIG. 5 shows graphs showing the fluorescence intensities of reaction solutions in which peptides YS were added to an Aβ25-35 aggregate. FIG. 5(A) shows the results from YS-11, and FIG. 5(B) shows the results from YS-16.
[FIG. 6] FIG. 6 shows electron micrographs showing the degrees of aggregation in reaction solutions in which peptides YS were added to an Aβ25-35 aggregate.
[FIG. 7] FIG. 7 shows graphs showing the alternation rates of mouse groups. FIG. 7(A) shows the result from coadministration of Aβ25-35 and a peptide YS, and FIG. 7(B) shows the result of administration of Aβ25-35 followed by administration of a peptide YS.

### Description of Embodiments

### <Amyloid-β Aggregation Inhibitor>

As described above, the amyloid-β aggregation inhibitor of the present invention is characterized by containing a peptide (A1) or (A2) below. The peptides (A1) and (A2) above are referred to collectively as a "peptide (A)", or a "peptide YS" or "aggregation inhibiting peptide YS". The aggregation inhibitor of the present invention is characterized by containing the peptide YS, and there is no particular limitation on the other configurations and conditions.
(A1) A peptide having an amino acid sequence of 5 or more consecutive amino acid residues in the amino acid sequence of SEQ ID NO: 1
   YKNMRETLVYLTHLDYDDTE (SEQ ID NO: 1)
(A2) A peptide having an amino acid sequence obtained by performing deletion, substitution, or addition of 1 or 2 amino acids on the amino acid sequence of (A1) above

It is sufficient that the length of the peptide YS (A1) is a length corresponding to 5 or more amino acid residues, and the length corresponds to, for example, 5 to 16 amino acid residues.

The peptide YS (A1) is, for example, a peptide having an amino acid sequence with 5 or more consecutive amino acid residues containing at least one of the fifth amino acid residue R (underlined R in Table 1) and the fifteenth amino acid residue D (underlined R in Table 1) in SEQ ID NO: 1, and preferably a peptide having an amino acid sequence with 11 or more consecutive amino acid residues containing both the fifth amino acid residue R (underlined) and the fifteenth amino acid residue D (underlined) in SEQ ID NO: 1. The peptide YS (A1) is more preferably a peptide having the fifth amino acid residue R (underlined) in SEQ ID NO: 1 at the N-terminus, or a peptide having the fifteenth amino acid residue D (underlined) in SEQ ID NO: 1 as the C-terminal amino acid residue. An example of such a peptide YS is, for example, a peptide having an amino acid sequence of SEQ ID NO: 2, 3, 4, or 5 illustrated in Table 1.

**[Table 1]**

| Peptide name | Sequence | | SEQ ID NO: |
|---|---|---|---|
| | YKNMRETLVYLTHLDYDDTE | | 1 |
| YS-5 | YKNMR | | 2 |
| YS-11 | | RETLVYLTHLD | 3 |
| YS-15 | YKNMRETLVYLTHLD | | 4 |
| YS-16 | | RETLVYLTHLDYDDTE | 5 |

As described above, the peptide (A2) has an amino acid sequence obtained by performing deletion, substitution, or addition of 1 or 2 amino acids on the amino acid sequence of the peptide (A1). In the case where the peptide YS-5, YS-11, YS-15, or YS-16 is used as the peptide (A1), the peptide (A2) has, for example, an amino acid sequence obtained by performing deletion, substitution, or addition of 1 or 2 amino acids on the amino acid sequence of the peptide YS-5, YS-11, YS-15, or YS-16.

The peptide YS may contain, for example, only D-amino acid residues, only L-amino acid residues, or both D-amino acid residues and L-amino acid residues, as the constitutional units.

The peptide YS may be, for example, a chemically modified peptide isoster. The chemical modification may be performed, for example, on all of the amino acid residues or some of the amino acid residues. There is no particular limitation on the type of chemical modification, and examples thereof include substitution of hydrogen atoms with halogen atoms, substitution with non-naturally occurring amino acids corresponding to naturally occurring amino acids, introduction of methyl groups, modification of amino acid side chains, cyclization of peptides, modification of carboxyl groups and/or amino groups, and amidation of terminal carboxyl groups.

The aggregation inhibitor of the present invention may contain, for example, only one, or any two, or three or more of the peptides YS-5, YS-11, YS-15, and YS-16.

In the present invention, aggregation of amyloid-β (also referred to as "Aβ" hereinafter) is, for example, aggregation of amyloid-β or fragment peptides thereof. In the description below, the meaning of aggregation of Aβ also includes the meaning of aggregation of the fragment peptides unless otherwise stated.

The peptide YS can inhibit aggregation of Aβ or fragment peptides thereof (also referred to as "Aβ fragments" hereinafter). In the present invention, aggregation inhibition is, for example, inhibition of aggregation of Aβ or the Aβ fragments, or inhibition through dissociation of Aβ aggregates or Aβ fragment aggregates that have already been formed. The peptide YS of the present invention can, for example, dissociate Aβ aggregates or Aβ fragment aggregates and inhibit aggregation itself. The dissociation of Aβ aggregates means, for example, breaking the aggregates up into monomeric components, i.e., Aβ, and the dissociation of Aβ fragment aggregates means, for example, breaking the aggregates up into monomeric components, i.e., Aβ fragments. In this case, the aggregation inhibitor of the present invention can be, for example, said to be an aggregation dissociating agent. The term "Aβ aggregates" in the description below is interchangeable with, for example, the term "Aβ fragment aggregates".

There is no particular limitation on the source of Aβ or the Aβ fragment whose aggregation is inhibited by the peptide YS, and examples thereof include a human and a non-human animal, which will be described later, with the source preferably being a human. The length of the full-length amino acid sequence of human Aβ is, for example, the length corresponding to 40 to 42 amino acid residues. The length of the full-length amino acid sequence of human amyloid-β depends on, for example, the site of enzymatic cleavage in the amyloid precursor (APP). The human amyloid-β constituted by 42 amino acid residues is registered, for example, in the database (PubChem) under accession number CID: 57339251 and is represented by SEQ ID NO: 6. The human amyloid-β constituted by 40 amino acid residues has a sequence from the first amino acid residue (D) at the N-terminus to the fortieth amino acid residue (V) in SEQ ID NO: 6, and the fortieth amino acid residue is the C-terminus.
DAEFRHDSGYEVHHQKLVFFAEDVGSNKGAIIGLMVGGVVIA (SEQ ID NO: 6)

The aggregation inhibitor of the present invention can be used to, for example, treat diseases that are caused by aggregation of Aβ (also referred to as "Aβ aggregation diseases" hereinafter). In the present invention, the meaning of treatment includes, for example, the meaning of prevention, inhibition of progression, and amelioration (alleviation). The meaning of the prevention includes, for example, the meaning of prevention of recurrence. The aggregation inhibitor of the present invention can inhibit the formation of the aggregate itself and is thus useful for prevention and inhibition of progression. Also, the aggregation inhibitor can dissociate the aggregate and is thus useful for inhibition of progression and amelioration (alleviation). There is no particular limitation on the amyloid-β aggregation diseases, and examples thereof include diseases that can be caused by the aggregation of amyloid-β, and specifically include memory impairment, cognitive dysfunction, Alzheimer's disease, and cerebral amyloid angiopathy.

The aggregation inhibitor of the present invention can inhibit, for example, cell death of neuronal cells caused by aggregation of Aβ. It is known that the cell death of neuronal cells plays a part in, for example, the development of the clinical state of Alzheimer's disease and the like. Accordingly, the aggregation inhibitor of the present invention can also exert a therapeutic effect on Aβ aggregation diseases such as Alzheimer's disease by inhibiting cell death of neuronal cells through Aβ aggregation inhibition.

The aggregation inhibitor of the present invention is also referred to as an aggregation inhibiting composition of the present invention. The aggregation inhibitor of the present invention contains the peptide YS as an active component. The active component contained in the aggregation inhibitor of the present invention may be constituted by only the peptide YS, or may further include other substances that exhibit an aggregation inhibiting effect. The aggregation inhibitor of the present invention may be, for example, a composition containing only the active component or a composition containing the active component and other additive components. There is no particular limitation on the additive components, and examples thereof include pharmacologically acceptable components. As for the additive components, reference can be made, for example, to the following descriptions regarding the pharmaceutical composition.

The aggregation inhibitor of the present invention can be used, for example, in environments in which Aβ or Aβ fragments are present or are presumed to be present. The aggregation inhibitor of the present invention, for example, can be added to a test subject. The test subject may be a non-living-being-derived test subject that does not contain cells and the like, or a living-being-derived test subject that is cells such as brain cells, tissue such as brain tissue, or an organism. In the case of the latter test subject, the addition of the aggregation inhibitor can be, for example, performed in vivo or in vitro. The cells and tissue may be sourced from a human or a non-human animal, for example, and the organism may be, for example, a human or a non-human animal. Examples of the non-human animal include mammalian animals such as mice, rats, rabbits, horses, sheep, cattle, and camels.

The aggregation inhibitor of the present invention may contain, for example, an additional peptide in addition to the peptide YS. The additional peptide may be, for example, in a form that is bonded to the peptide YS. The additional peptide may have, for example, a DDS ability to the site in which Aβ, Aβ fragments, Aβ aggregates, or Aβ fragment aggregates are present. Furthermore, the additional peptide may be, for example, a signal peptide that is bonded to the peptide YS at the time of administration to an organism and is cleaved from the peptide YS by an enzyme or the like in the organism after administration to the organism.

The aggregation inhibitor of the present invention can be used, for example, also as a neuronal cell death inhibitor or pharmaceutical composition for an Aβ aggregation disease of the present invention, which will be described later. Furthermore, the aggregation inhibitor of the present invention can be used, for example, in a method for inhibiting aggregation of AB, a method for inhibiting neuronal cell death, a method for treating an Aβ aggregation disease, and the like of the present invention, which will be described later. As for the aggregation inhibitor of the present invention, reference can be made to the following descriptions regarding the neuronal cell death inhibitor, the pharmaceutical composition for an Aβ aggregation disease, the method for inhibiting aggregation of A, the method for inhibiting neuronal cell death, and the method for treating an amyloid-β aggregation disease of the present invention, which will be described later.

### <Neuronal Cell Death Inhibitor>

As described above, the neuronal cell death inhibitor of the present invention is characterized by containing the peptide (A) (the peptide YS), namely the peptide (A1) or (A2). The neuronal cell death inhibitor of the present invention is characterized by containing the peptide YS, and there is no particular limitation on the other configurations and conditions. As for the neuronal cell death inhibitor, reference can be made to the foregoing descriptions regarding the Aβ aggregation inhibitor of the present invention.

The peptide YS of the present invention can inhibit neuronal cell death. Specifically, the peptide YS of the present invention can inhibit, for example, cell death of neuronal cells caused by aggregation of Aβ. As described above, it is known that the cell death of neuronal cells plays a part in, for example, the development of the clinical state of Alzheimer's disease and the like. Accordingly, through inhibition of aggregation of Aβ, the neuronal cell death inhibitor of the present invention can inhibit, for example, neuronal cell death induced by aggregation of Aβ. Furthermore, the neuronal cell death inhibitor can also exert a therapeutic effect on Aβ aggregation diseases (amyloid-β aggregation disease) such as Alzheimer's disease by inhibiting neuronal cell death in this manner.

<Pharmaceutical Composition for Amyloid-β Aggregation Disease>

As described above, the pharmaceutical composition for an amyloid-β aggregation disease (also referred to as a "pharmaceutical composition" hereinafter) of the present invention is characterized by containing the peptide (A) (the peptide YS), namely the peptide (A1) or (A2). The pharmaceutical composition of the present invention is characterized by containing the peptide YS, and there is no particular limitation on the other configurations and conditions. As for the pharmaceutical composition, reference can be made to the foregoing descriptions regarding the Aβ aggregation inhibitor and the neuronal cell death inhibitor of the present invention.

The pharmaceutical composition of the present invention contains the peptide YS as an active component. The active component contained in the pharmaceutical composition of the present invention may be constituted by only the peptide YS, or may further include other active components on the Aβ aggregation diseases. The other active components may be, for example, active components that inhibit the formation of aggregates, active components that dissociate the aggregates that have been formed, or active components that degrade the aggregates. The degradation of aggregates may be, for example, degradation through cleavage of the aggregates using hydrolytic activity or the like.

The pharmaceutical composition of the present invention may be, for example, a composition containing only the active component or a composition containing the active component and other additive components. There is no particular limitation on the additive components, and examples thereof include pharmacologically acceptable components. The additive components may be, for example, selected as appropriate according to the administration method, the administration site, the dosage form, and the like of the pharmaceutical composition of the present invention.

There is no particular limitation on the administration method of the pharmaceutical composition of the present invention, and examples thereof include parenteral administration and oral administration.

Examples of parenteral administration include administration to an affected site, intravenous administration, subcutaneous administration, intradermal administration, nasal administration, and dermal administration. The administration can be performed using, for example, drip injection, injection, or the like. As for the administration site of the parenteral administration, the pharmaceutical composition may be, for example, directly administered to a treatment site or indirectly administered to a treatment site. In the latter case, the administration site is, for example, a site from which the active component of the pharmaceutical composition of the present invention can be delivered to the treatment site. In many cases, Aβ aggregation diseases are caused by, for example, aggregation of Aβ in the brain, as in the case of memory impairment, cognitive dysfunction, Alzheimer's disease, and the like mentioned above. Thus, the treatment site is, for example, the brain, and the administration method is preferably direct administration to the brain, for example, by injection, nasal administration, or the like.

There is no particular limitation on the dosage form of the pharmaceutical composition of the present invention, and the dosage form may be selected as appropriate according to the administration method. The dosage form of the pharmaceutical composition of the present invention at the time of administration is, for example, liquid, cream, gel, powder, or the like. Furthermore, the dosage form of the pharmaceutical composition of the present invention before administration, specifically the dosage form during the distribution process, may be, for example, the same as or different from the dosage form at the time of administration. In the latter case, the dosage form may be, for example, a form that allows a pharmacist, a nurse, a physician (a doctor), or the like at the time of administration to prepare the pharmaceutical composition into a dosage form for use at the time of administration. The dosage form before administration may be, for example, a solid such as a powder or granules, a concentrated liquid, or the like.

As described above, the additive components to the pharmaceutical composition of the present invention may be selected as appropriate according to the administration method, the dosage form, and the like, and examples thereof include a solvent, a diluent, an excipient, and a carrier. The solvent may be, for example, an aqueous solvent such as water, a saline solution, an isotonic solution, or a buffer solution, an oil solvent such as soybean oil, or an emulsion solvent, which is a mixed solution of the aqueous solvent and the oil solvent. The pharmaceutical composition of the present invention may contain, for example, alcohols, polyalcohols, surfactants, and the like, as the additive components. Furthermore, the pharmaceutical composition of the present invention may contain, for example, a DDS agent for effectively delivering the active component to the treatment site. The pharmaceutical composition of the present invention may be, for example, in a form containing a carrier in which the active component is encapsulated. The carrier may be, for example, nanoparticles of polymers or the like. The form in which the active component is encapsulated in this manner can maintain the stability of the active component, and also allow the carrier to function, for example, as a DDS. In this case, for example, the pharmaceutical composition of the present invention is preferably used for administration, for example, by intravenous injection or the like.

Examples of the administration target (test subject) of the pharmaceutical composition of the present invention include a human and a non-human animal described above. There is no particular limitation on the administration conditions of the pharmaceutical composition of the present invention, and the administration conditions may be determined as appropriate according to the species, the age, the weight, the sex, the presence or absence of Aβ aggregation diseases, the degree of progression, and the like. If the administration target is an adult human male weighing 70 kg, the administration conditions of the pharmaceutical composition of the present invention are, for example, such that the amount of the peptide YS per administration is 0.002 to 400 mg, the administration frequency is once to three times a day, and the administration is performed with an interval of 1 to 10 days.

In this specification, as described above, the meaning of treatment includes, for example, the meaning of prevention, inhibition of progression, and amelioration (alleviation). The pharmaceutical composition of the present invention may be used, for example, for any one or more of these purposes.

### <Method for Inhibiting Aggregation of Amyloid-β>

As described above, the method for inhibiting aggregation of amyloid-β of the present invention is characterized by including adding the Aβ aggregation inhibitor of the present invention (i.e., adding the peptide YS as an active component) to a test subject. The inhibition method of the present invention is characterized by using the aggregation inhibitor of the present invention, and there is no limitation on the other steps, conditions, or the like.

As for the addition of the aggregation inhibitor of the present invention to the test subject, reference can be made to the foregoing descriptions regarding the aggregation inhibitor and the pharmaceutical composition of the present invention. It is preferable that the aggregation inhibition method of the present invention further includes, for example, performing incubation, after adding the aggregation inhibitor of the present invention to the test subject. If the test subject is the non-living being, the incubation temperature is, for example, from room temperature to 37°C, the incubation time is from 4 to 72 hours, and the pH is from 6.5 to 8. Also, if the test subject is the cell or tissue, the incubation temperature is, for example, from room temperature to 37°C, the incubation time is from 1 to 7 days, and the pH is from 6.5 to 8.

### <Method for Inhibiting Neuronal Cell Death>

As described above, the method for inhibiting neuronal cell death of the present invention is characterized by including adding the neuronal cell death inhibitor of the present invention (i.e., adding the peptide YS as an active component) to a test subject. The method for inhibiting neuronal cell death of the present invention is characterized by using the neuronal cell death inhibitor of the present invention, and there is no limitation on the other steps, conditions, or the like.

As for the addition of the neuronal cell death inhibitor of the present invention to the test subject, reference can be made to the foregoing descriptions regarding the aggregation inhibitor, the pharmaceutical composition, and the method for inhibiting aggregation of Aβ of the present invention. It is preferable that the method for inhibiting neuronal cell death of the present invention further includes, for example, performing incubation, after adding the neuronal cell death inhibitor of the present invention to the test subject. If the test subject is the non-living being, the incubation temperature is, for example, from room temperature to 37°C, the incubation time is from 4 to 72 hours, and the pH is from 6.5 to 8. Also, if the test subject is the cell or tissue, the incubation temperature is, for example, from room temperature to 37°C, the incubation time is from 1 to 7 days, and the pH is from 6.5 to 8.

### <Method for Treating Amyloid-β Aggregation Disease>

As described above, the method for treating an amyloid-β aggregation disease of the present invention is characterized by including administering the Aβ aggregation inhibitor of the present invention or the pharmaceutical composition of the present invention (i.e., administering the peptide YS as an active component) to a test subject. The treating method of the present invention is characterized by using the Aβ aggregation inhibitor of the present invention or the pharmaceutical composition of the present invention (i.e., using the peptide YS as the active component), and there is no limitation on the other steps, conditions, or the like.

Examples of the administration target (test subject) of the amyloid-β aggregation inhibitor of the present invention include a human and a non-human animal described above, with the administration target preferably being a human. As for the addition of the aggregation inhibitor of the present invention to the test subject, reference can be made to the foregoing descriptions regarding the aggregation inhibitor and the pharmaceutical composition of the present invention.

### <Use of Peptide YS>

The peptide of the present invention is the peptide YS used to inhibit aggregation of Aβ or neuronal cell death, namely the peptide (A1) or (A2). Also, the peptide of the present invention is the peptide YS used to treat an Aβ aggregation disease caused by Aβ aggregation, namely the peptide (A1) or (A2).

The peptide of the present invention is the peptide YS used to produce the Aβ aggregation inhibitor or the neuronal cell death inhibitor, namely the peptide (A1) or (A2). Also, the peptide of the present invention is the peptide YS used to produce a medicine for an Aβ aggregation disease caused by Aβ aggregation, namely the peptide (A1) or (A2).

### Examples

### [Example 1]

The ability of the aggregation inhibiting peptide YS to inhibit Aβ aggregation was confirmed.

### (1) Confirming of Ability to Inhibit Aβ Aggregation

The peptides YS-11, YS-15, and YS-16 were used as the peptides YS of the examples. Each of the peptides was dissolved in MilliQ water to prepare three types of 1-mg/mL YS solutions.

An Aβ-derived fragment peptide was used for aggregation of Aβ. Aβ25-35, which has high aggregability, was selected as the fragment peptide. The AB25-35 is a peptide (SEQ ID NO: 7: GSNKGAIIGLM) constituted by 11 amino acid residues consisting of the twenty-fifth to thirty-fifth amino acid residues in the full length sequence of human-derived Aβ (SEQ ID NO: 6). The Aβ25-35 was dissolved in MilliQ water to prepare a 1-mmol/L Aβ25-35 solution.

The fluorescent dye thioflavin T (ThT) binds to aggregates and emits strong fluorescence upon binding, and thus the increase or inhibition of aggregation can be determined by measuring the fluorescence intensity. Thus, the inhibition of aggregation of the Aβ25-35 by each peptide YS was checked using ThT.

Specifically, a reaction solution with the following composition was prepared and dispensed into wells such that the amount of the reaction solution per well was 200 pL. After the reaction solution was incubated at 37°C for a predetermined period of time (0 or 4 hours), 10 pL of 2-mmol/L ThT (reagent name "Thioflavine T", manufactured by FUJIFILM Wako Pure Chemical Corporation) was added to each well, and the fluorescence intensity was measured using a measurement device (product name "Cytation5", manufactured by BioTek) (n=3). The measurement wavelengths were set such that the excitation wavelength (ex) was 444 nm and the fluorescence wavelength (em) was 480 nm. As a control, a reaction solution to which water had been added instead of the peptide YS was also measured in the same way.

**[Table 2]**

| | Amount | Final conc. |
|---|---|---|
| 1 mM Peptide YS | 200 uL | 0.1 mM |
| 1 mM Aβ25-35 | 200 uL | 0.1 mM |
| 10 × PBS | 200 uL | |
| MilliQ | 14000 uL | |
| Total | 2000 uL | - |

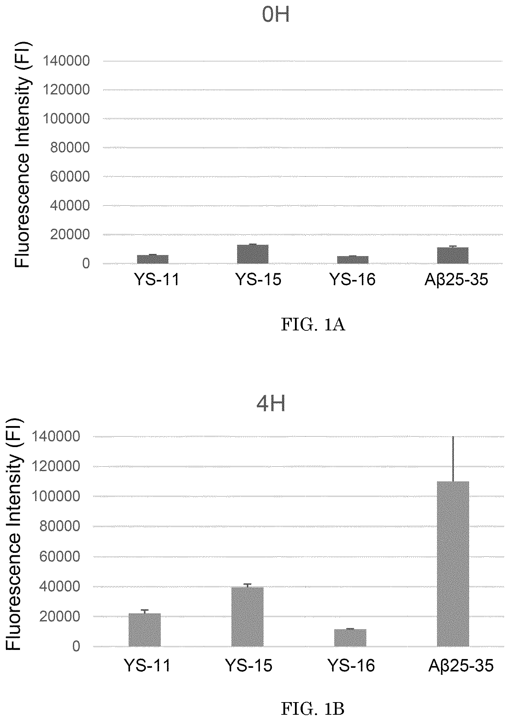

FIG. 1 shows the results. FIG. 1 shows graphs showing the fluorescence intensities of the reaction solutions to which the peptides YS had been added. FIG. 1(A) shows the results when the incubation time was 0 hours, and FIG. 1(B) shows the results when the incubation time was 4 hours. In FIG. 1, the vertical axes indicate the fluorescence intensity (unit: Fluorescence Intensity (FI)). As shown in FIG. 1(B), in all the cases of the peptides YS-11, YS-15, and YS-16, the fluorescence intensity was significantly lower after the incubation for 4 hours compared with the control (Ab25-35) to which the peptide YS had not been added. In particular, in the cases of the YS-11 and the YS-16, the fluorescence intensity was considerably lower. It was found from these results that the peptides YS can inhibit aggregation of Aβ (formation of Aβ aggregates).

### (2) IC50 of YS Peptide against Aβ25-35

Reaction solutions with different compositions, namely those with the following compositions (A, B, C, and D), were prepared and dispensed into wells such that the amount of the reaction solution per well was 200 µL. The concentration (X mmol/L) of the peptide YS (YS-11 or YS-16) in the YS solution to be added to the reaction solutions was set to 0.01, 0.05, 0.1, 0.5, and 1 mmol/L. The reaction solutions were incubated at 37°C, and 10 pL of 2-mmol/L ThT (reagent name "Thioflavine T", manufactured by FUJIFILM Wako Pure Chemical Corporation) was added to each well 2 hours after the start of the incubation. Then, the fluorescence intensity was measured using the measurement device (n=3). The measurement wavelengths were set such that the excitation wavelength (ex) was 444 nm and the fluorescence wavelength (em) was 480 nm.

**[Table 3]**

| | Amount | | | |
|---|---|---|---|---|
| | A | B | C | D |
| X mM YS-11 or YS-16 | 100 uL | 100 uL | - | - |
| 1 mM Aβ25-35 | 100 uL | - | 100 uL | - |
| 10 × PBS | 100 uL | 100 uL | 100 uL | 100 uL |
| MilliQ | 700 uL | 800 uL | 800 uL | 900 uL |
| Total | 1000 uL | 1000 uL | 1000 uL | 1000 uL |

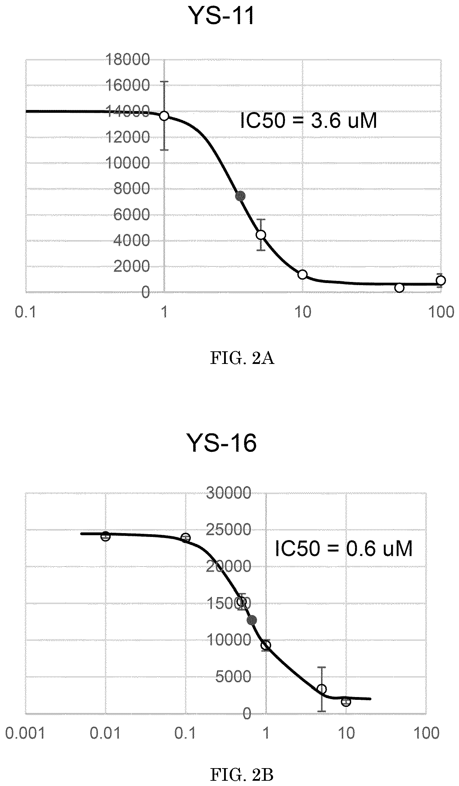

Then, blank correction was performed by subtracting a fluorescence intensity FI_{B} of the reaction solution B, to which the peptide YS had been added and the Aβ25-35 had not been added, from a fluorescence intensity FI_{A} of the reaction solution A, to which the peptide YS had been added and the Aβ25-35 had been added, and subtracting a fluorescence intensity FI_{D} of the reaction solution D, to which the peptide YS had not been added and the Aβ25-35 had not been added, from a fluorescence intensity FI_{C} of the reaction solution C, to which the peptide YS had not been added and the Aβ25-35 had been added. FIG. 2 shows the results. In FIG. 2, the vertical axes indicate the fluorescence intensity (unit: FI), and the horizontal axes indicate the concentration of the peptide YS (unit: pmol/L). FIG. 2(A) shows the result from the YS-11, and FIG. 2(B) shows the result from the YS-16. As shown in FIG. 2, the IC50 of the YS-11 was 3.6 pmol/L, and the IC50 of the YS-16 was 0.6 pmol/L.

### (3) Checking of Aggregation Inhibition by Peptide YS Using Electron Microscope

A reaction solution with the following composition was prepared in a 1.5-mL tube (manufactured by Eppendorf). After incubating at 37°C for 4 hours, the reaction solution was centrifuged (12,000 rpm, 10 minutes), and a precipitate was collected. The collected precipitate was washed as follows: 200 pL of MilliQ water was added to the precipitate and the resultant mixture was centrifuged again (12,000 rpm, 10 minutes). This washing treatment was performed three times. Then, 100 pL of a 1% phosphotungstic acid solution was added to the collected precipitate. After the resultant mixture was left to stand for 5 minutes at room temperature, the precipitate was collected through centrifugation (12,000 rpm, 10 minutes), was washed by adding 200 µL of MilliQ water thereto, and was collected through centrifugation (12,000 rpm, 10 minutes). 100 pL of MilliQ water was added to the precipitate, and a suspension was formed using a Vortex mixer. The suspension was added to a glass slide and dried, followed by observation under a scanning electron microscope.

**[Table 4]**

| | Amount | | Final conc. |
|---|---|---|---|
| 1 mM YS-11 or YS-16 | 100 uL | - | 0.1 mM |
| 1 mM Aβ25-35 | 100 uL | 100 uL | 0.1 mM |
| 10 × PBS | 100 uL | 100 uL | |
| MilliQ | 700 uL | 800 uL | |
| Total | 1000 uL | 1000 uL | - |

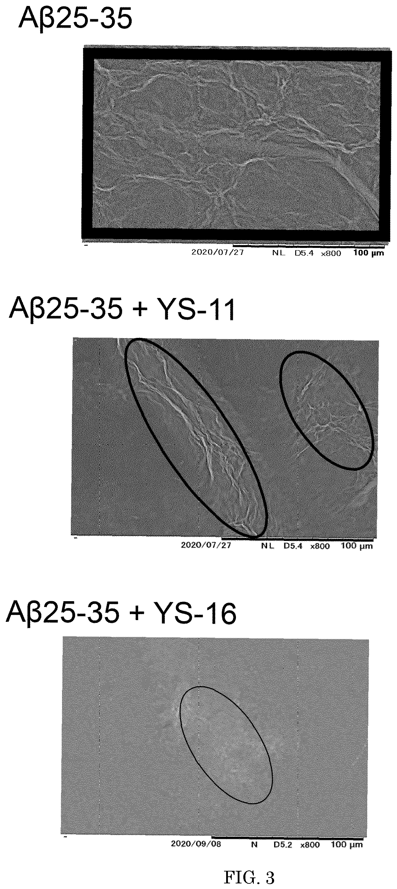

FIG. 3 shows the results. In FIG. 3, the upper panel shows the result when the peptide YS was not added, the middle panel shows the result when the peptide YS-11 was added, and the lower panel shows the result when the peptide YS-16 was added (800-fold). In the photographs in FIG. 3, a region in which fibers were observed is framed or circled by a line. The thickness of the line corresponds to the amount of fibers in the region, and the larger the amount of fibers is, the thicker the line is. As shown in the upper panel, when the peptide YS was not added and the Ab25-35 was added alone, a large number of aggregated fibers were observed in the entire field of view. Meanwhile, as shown in the middle panel, when the Ab25-35 and the YS-11 coexisted, the amount of a fibrous material decreased very significantly, and the formation of aggregates was inhibited compared with the case where the peptide YS was not added. Moreover, as shown in the lower panel, when the Ab25-35 and the YS-16 coexisted, substantially no fibrous material was observed, and the formation of aggregates was significantly inhibited.

### [Example 2]

Dissociation of aggregates was checked to evaluate the aggregation inhibiting ability of the peptide YS.

### (1) Checking of Ability to Dissociate Aβ Aggregates

First, a reaction solution with the following composition was prepared and incubated at 37°C for 18 hours to form aggregates of the Aβ25-35 in the reaction solution. 100 pL of a 1-mmol/L peptide YS (YS-5 or YS-16) was further added to the reaction solution, and the resultant mixture was incubated at 37°C for 5 days. After the incubation, the reaction solution was dispensed into wells such that the amount of the reaction solution per well was 200 pL. Furthermore, 10 pL of 2-mmol/L ThT was added to each well, and the fluorescence intensity was measured using the measurement device (n=3). The measurement wavelengths were set such that the excitation wavelength (ex) was 444 nm and the fluorescence wavelength (em) was 480 nm. As a control, a reaction solution to which water had been added instead of the peptide YS was also measured in the same way.

**[Table 5]**

| | Amount | Final conc. |
|---|---|---|
| 1 mM Aβ25-35 | 100 uL | 0.1 mM |
| 10 × PBS | 100 uL | |
| MilliQ | 700 uL | |
| Total | 1000 uL | - |

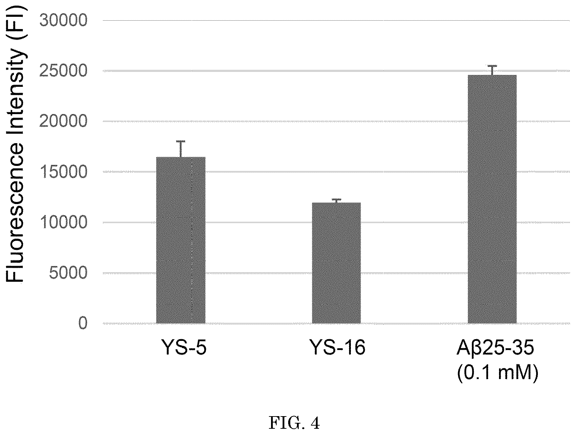

FIG. 4 shows the results. FIG. 4 is a graph showing the fluorescence intensities of the reaction solutions to which the peptide YS was added. In FIG. 4, the vertical axis indicates the fluorescence intensity (unit: Fluorescence Intensity (FI)). As shown in FIG. 4, the fluorescence intensity decreased due to the addition of the peptide YS compared with the control (Aβ25-35) to which the peptide YS had not been added. In particular, in the case of using the peptide YS-16, the fluorescence intensity significantly decreased to 50% of that in the case of the control (Aβ25-35). It was found from these results that the peptide YS can dissociate Aβ aggregates, that is, inhibit aggregation of Aβ. In particular, it can also be said that the YS-16 can dissociate 50% of control aggregates.

### (2) Relationship between Peptide YS Concentration and Ability to Dissociate Aβ Aggregates

First, a reaction solution with the following composition was prepared, was dispensed into wells such that the amount of the reaction solution per well was 200 pL, and was incubated at 37°C for 18 hours to form aggregates of the Aβ25-35 in the reaction solution. 100 pL of an X-mmol/L peptide YS solution (YS-11 or YS-16) was further added to the reaction solution, and the resultant mixture was incubated at 37°C for 5 days. The concentration (X mmol/L) of the peptide YS in the peptide YS solutions was set to 0.01, 0.05, 0.1, 0.5, and 1 mmol/Lmmol/L. After the incubation, 10 pL of 2-mmol/L ThT was added to each well, and the fluorescence intensity was measured using the measurement device (n=3). The measurement wavelengths were set such that the excitation wavelength (ex) was 444 nm and the fluorescence wavelength (em) was 480 nm. As a control, a reaction solution (Aβ25-35) to which water had been added instead of the peptide YS was also measured in the same way.

**[Table 6]**

| | Amount | Final conc. |
|---|---|---|
| 1 mM Aβ25-35 | 100 uL | 0.1 mM |
| 10 × PBS | 100 uL | |
| MilliQ | 700 uL | |
| Total | 1000 uL | - |

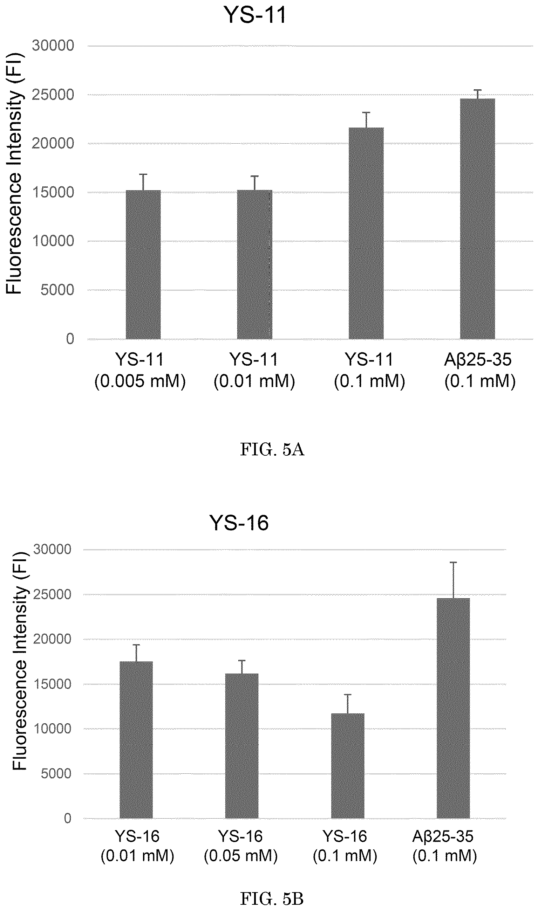

FIG. 5 shows the results. FIG. 5 shows graphs showing the fluorescence intensities of the reaction solutions to which peptide YS was added. FIG. 5(A) shows the results from the YS-11, and FIG. 5(B) shows the results from the YS-16. In FIG. 5, the vertical axes indicate the fluorescence intensity (unit: Fluorescence Intensity (FI)). As shown in FIG. 5(A), the peptide YS-11 exhibited an excellent dissociating ability when used within a final concentration range of 0.005 to 0.1 mmol/L in the reaction solution. Also, as shown in FIG. 5(B), the peptide YS-16 exhibited an excellent dissociating ability in a concentration-dependent manner when used within a final concentration range of 0.01 to 0.1 mmol/L in the reaction solution. In particular, when the aggregates in the control (Aβ25-35) is taken as 100%, 48% of the aggregates could be dissociated by adding the YS-16 at a final concentration of 0.1 mmol/L.

### (3) Checking of Dissociation of Aggregates by YS Peptide Using Electron Microscope

A reaction solution with the following composition was prepared in a 1.5-mL tube (manufactured by Eppendorf) and incubated at 37°C for 18 hours to form aggregates of the Aβ25-35. Then, 100 pL of a peptide YS solution at a predetermined concentration was added to the reaction solution, and the resultant mixture was further incubated at 37°C for 5 days. A 0.05-mmol/L YS-11 solution, a 1-mmol/L YS-16 solution, and a 1-mmol/L YS-6 solution were used as the peptide YS solution, and MilliQ water was used instead of the peptide YS solution as a control. After the incubation, centrifugation (12,000 rpm, 10 minutes) was performed to collect a precipitate. The precipitate was treated in the same manner as in Example 1(3) above, and was then observed under a scanning electron microscope.

**[Table 7]**

| | Amount | Final conc. |
|---|---|---|
| 1 mM Aβ25-35 | 100 uL | 0.1 mM |
| 10 × PBS | 100 uL | |
| MilliQ | 700 uL | |
| Total | 1000 uL | - |

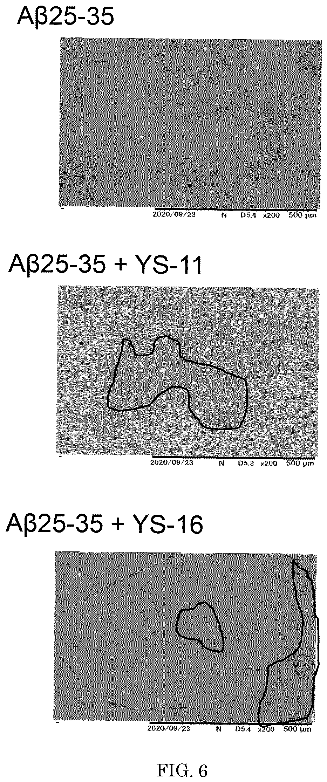

FIG. 6 shows the results. In FIG. 6, the upper panel shows the result when the peptide YS was not added, the middle panel shows the result when the peptide YS-11 was added, and the lower panel shows the result from the peptide YS-16 (200-fold). In this example, aggregates of the Aβ25-35 were formed in advance, and then dissociation of the aggregates caused by the peptide YS was checked. As shown in the upper panel, when the peptide YS was not added and the Ab25-35 was added alone, a large number of aggregated fibers were observed in the entire field of view. Meanwhile, as shown in the lower panel, when the Ab25-35 and the YS-16 coexisted, a decrease in the amount of fibrous aggregates was observed, particularly in the region surrounded by a line. Also, as shown in the middle panel, when the Ab25-35 and the YS-11 coexisted, a further decrease in the amount of fibrous aggregates was observed, particularly in the region surrounded by a line.

### [Example 3]

The aggregation inhibiting peptide was further administered to mice to which the Aβ25-35 had been administered, and then the effects of the aggregation inhibiting peptide was checked.

The Aβ25-35 was dissolved in a saline solution to prepare a 3-mmol/L Aβ25-35 solution. The Aβ25-35 and the YS-11 were dissolved in a saline solution to prepare a mixed solution containing the Aβ25-35 at a concentration of 3 mmol/L and the YS-11 at a concentration of 3 mmol/L. The YS-11 was dissolved in a saline solution to prepare a 10-mg/mL YS-11 solution. A saline solution (Saline) was used as a control.

ICR mice (6-week old) were prepared and divided into an Aβ25-35 administration group (n=15), a control (Saline) administration group (n=15), a mixed solution administration group (n=6), and a peptide YS administration group (n=7).

The administration schedule was as follows: ventricular administration was performed on Day 1, and nasal administration was performed on Day 14, Day 17, Day 20, and Day 23. A microsyringe was used for the ventricular administration, and a Pipetman was used for the nasal administration. Specifically, the administration was performed on each administration group as follows.
(1) Aβ25-35 administration group
   Day 1: 3 pL of Aβ25-35 solution (9 nmol of Aβ25-35)
   Day 14, Day 17, Day 20, Day 23: not administered
(2) Control administration group (Saline)
   Day 1: 3 pL of Saline
   Day 14: 3 pL of Saline
   Day 17: 3 pL of Saline
   Day 20: 3 pL of Saline
   Day 23: 3 pL of Saline
(3) Mixed solution administration group
   Day 1: 3 pL of mixed solution (9 nmol of Aβ25-35, 9 nmol of YS-11)
   Day 14: 3 pL of Saline
   Day 17: 3 pL of Saline
   Day 20: 3 pL of Saline
   Day 23: 3 pL of Saline
(4) Peptide YS administration group
   Day 1: 3 pL of Aβ25-35 solution (9 nmol of Aβ25-35)
   Day 14: 10 pL of YS-11 solution (74 nmol of YS-11)
   Day 17: 10 pL of YS-11 solution (74 nmol of YS-11)
   Day 20: 10 pL of YS-11 solution (74 nmol of YS-11)
   Day 23: 10 pL of YS-11 solution (74 nmol of YS-11)

Then, on Day 14 and Day 28, the short-term memory was evaluated using a Y-maze. The Y-maze test was conducted in a general manner under the following conditions. That is to say, mice were placed such that their noses faced the wall at an end of one particular arm among the three arms. The mice were allowed to move freely for 10 minutes, and their behavior was captured on a monitor of an analyzer (product name: Time YM1, manufactured by O'hara & Co., Ltd.) to calculate the alternation rate (%). A relatively high alternation rate means an excellent short-term memory, and a relatively low alternation rate means a poor short-term memory.

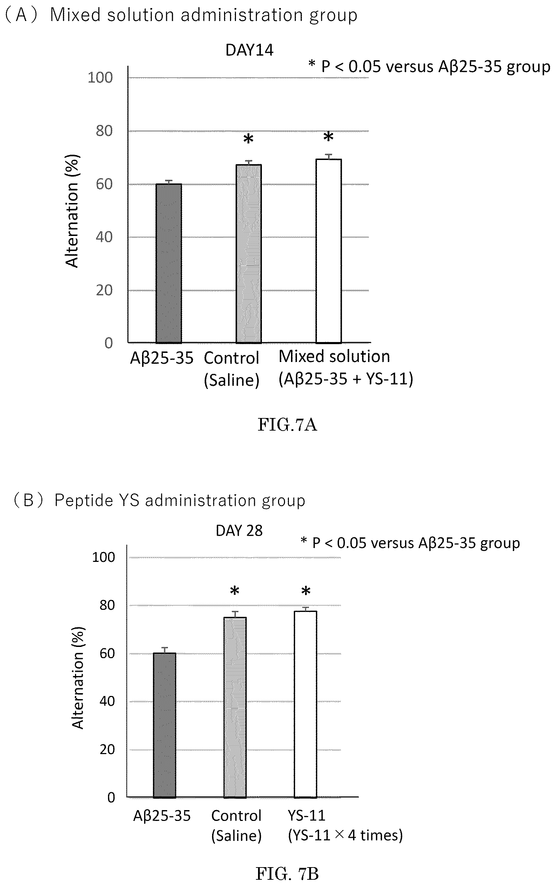

FIG. 7 shows the results of the alternation rate (%) calculated using the Y-maze. In FIG. 7(A), the results from the Aβ25-35 administration group (Aβ25-35), the control administration group (Control), and the mixed solution administration group (Mixed solution) were compared. In FIG. 7(B), the results from the Aβ25-35 administration group (Aβ25-35), the control administration group (Control), and the peptide YS administration group (YS-11) were compared. In FIG. 7, the vertical axes indicate the alternation rate (%).

First, as shown in FIGS. 7(A) and 7(B), the alternation rate of the control group (Control) to which the Aβ25-35 had not been administered was about 70 to 75% on Day 14 and Day 28, and did not change over time. On the contrary, on both Day 14 and Day 28, the alternation rate of the Aβ25-35 administration group (Aβ25-35) to which only the Aβ25-35 had been administered was lower than that of the control group (Control). That is to say, it is understood that the alternation rate decreased due to the aggregation of the Aβ25-35.

As shown in FIG. 7(A), on Day 14, the alternation rate of the mixed solution administration group (Mixed solution) to which the Aβ25-35 and the YS-11 had been simultaneously administered was higher than that of the Aβ25-35 administration group (Aβ25-35) to which only the Aβ25-35 had been administered, and was as high as that of the control group to which the Aβ25-35 had not been administered. Since the Aβ25-35 and the YS-11 were simultaneously administered to the mixed solution administration group, it is understood that the formation of Aβ25-35 aggregates was inhibited, thus making it possible to suppress a decrease in the alternation rate caused by the aggregation of the Aβ25-35.

In the peptide YS administration group (YS-11), the Aβ25-35 was ventricularly administered on Day 1, and the short-term memory was evaluated using the Y-maze on Day 14. The result therefrom was similar to that from the Aβ25-35 administration group (Aβ25-35) to which only the Aβ25-35 had been administered, which is shown in FIG. 7(A). That is to say, on Day 14, the alternation rate of the peptide YS administration group (YS-11) was lower than that of the control group (Control) (not illustrated). Thereafter, the peptide YS-11 was nasally administered four times in total on Day 14, Day 17, Day 20, and Day 23, and the short-term memory was evaluated using the Y-maze again on Day 28. FIG. 7(B) shows the result. On Day 14, the ventricular administration of the Aβ25-35 lowered the alternation rate compared with the control group. However, as shown in FIG. 7(B), as a result of administering the peptide YS-11 four times from Day 14, the alternation rate on Day 28 was higher than that of the Aβ25-35 administration group (Aβ25-35) to which only the Aβ25-35 had been administered, and was as high as that of the control group to which the Aβ25-35 had not been administered. That is to say, the alternation rate recovered. Since the YS-11 was administered to the peptide YS administration group after the administration of the Aβ25-35, it is understood that the YS-11 dissociated aggregates formed from the previously administered Aβ25-35, thus making it possible to suppress a decrease in the alternation rate caused by the aggregation of the Aβ25-35.

As described above, the administration of the Aβ25-35 to the brain of a mouse results in aggregation of the Aβ25-35, leading to the deterioration of the short-term cognitive function, which is the symptom of Alzheimer's disease, but it is found that the administration of the peptide YS makes it possible to suppress the deterioration of the short-term cognitive function.

In the description above, the present invention was described by way of embodiments and examples, but the invention is not limited to the foregoing embodiments and examples. Various changes that can be understood by those skilled in the art can be made to the configuration and details of the invention within the scope of the invention.

This application claims the benefit of priority from Japanese Patent Application No. 2021-80513, filed on May 11, 2021, the entire contents of which are incorporated herein by reference.

### Industrial Applicability

The amyloid-β aggregation inhibitor of the present invention can inhibit the aggregation of amyloid-β caused by intermolecular association. This enables treatment, e.g., prevention, inhibition of progression, and amelioration, of amyloid aggregation diseases such as Alzheimer's disease caused by aggregation of amyloid-β.

## Claims

1. An amyloid-β aggregation inhibitor comprising a peptide (A1) or (A2) below:
(A1) a peptide having an amino acid sequence of 5 or more consecutive amino acid residues in an amino acid sequence of SEQ ID NO: 1,
YKNMRETLVYLTHLDYDDTE (SEQ ID NO: 1);
(A2) a peptide having an amino acid sequence obtained by performing deletion, substitution, or addition of 1 or 2 amino acids on the amino acid sequence of (A1) above.

2. The aggregation inhibitor according to claim 1, wherein the peptide (A1) includes 5 to 16 amino acid residues.

3. The aggregation inhibitor according to claim 1 or 2, wherein the peptide (A1) is a peptide having an amino acid sequence with 5 or more consecutive amino acid residues containing at least one of the fifth amino acid residue R and the fifteenth amino acid residue D in SEQ ID NO: 1.

4. The aggregation inhibitor according to any one of claims 1 to 3, wherein the peptide (A1) is a peptide having an amino acid sequence with 11 or more consecutive amino acid residues containing both the fifth amino acid residue R and the fifteenth amino acid residue D in SEQ ID NO: 1.

5. The aggregation inhibitor according to any one of claims 1 to 4, wherein the peptide (A1) is a peptide having the fifth amino acid residue R in SEQ ID NO: 1 at an N-terminus, or a peptide having the fifteenth amino acid residue D in SEQ ID NO: 1 at a C-terminus.

6. The aggregation inhibitor according to any one of claims 1 to 3, wherein the peptide (A1) is a peptide having an amino acid sequence of SEQ ID NO: 2, 3, 4, or 5:
YKNMR (SEQ ID NO: 2);
RETLVYLTHLD (SEQ ID NO: 3);
YKNMRETLVYLTHLD (SEQ ID NO: 4);
RETLVYLTHLDYDDTE (SEQ ID NO: 5).

7. A pharmaceutical composition for an amyloid-β aggregation disease caused by amyloid-β aggregation, comprising a peptide (A1) or (A2) below:
(A1) a peptide having an amino acid sequence of 5 or more consecutive amino acid residues in an amino acid sequence of SEQ ID NO: 1,
YKNMRETLVYLTHLDYDDTE (SEQ ID NO: 1);
(A2) a peptide having an amino acid sequence obtained by performing deletion, substitution, or addition of 1 or 2 amino acids on the amino acid sequence of (A1) above.

8. The pharmaceutical composition according to claim 7, wherein the peptide (A1) includes 5 to 16 amino acid residues.

9. The pharmaceutical composition according to claim 6 or 7, wherein the peptide (A1) is a peptide having an amino acid sequence with 5 or more consecutive amino acid residues containing at least one of the fifth amino acid residue R and the fifteenth amino acid residue D in SEQ ID NO: 1.

10. The pharmaceutical composition according to any one of claims 6 to 8, wherein the peptide (A1) is a peptide having an amino acid sequence of SEQ ID NO: 2, 3, 4, or 5:
YKNMR (SEQ ID NO: 2);
RETLVYLTHLD (SEQ ID NO: 3);
YKNMRETLVYLTHLD (SEQ ID NO: 4);
RETLVYLTHLDYDDTE (SEQ ID NO: 5).

11. The pharmaceutical composition according to any one of claims 6 to 10, wherein the amyloid-β aggregation disease is at least one selected from the group consisting of memory impairment, cognitive dysfunction, Alzheimer's disease, and cerebral amyloid angiopathy.

12. A method for inhibiting aggregation of amyloid-β, comprising adding the amyloid-β aggregation inhibitor according to any one of claims 1 to 6 to a test subject.

13. The aggregation inhibition method according to claim 12, wherein the aggregation inhibitor is added in vivo or in vitro.

14. The aggregation inhibition method according to claim 12 or 13, wherein the aggregation inhibitor is added to a human or a non-human animal.

15. A method for treating an amyloid-β aggregation disease caused by amyloid-β aggregation, comprising administering the amyloid-β aggregation inhibitor according to any one of claims 1 to 6 to a test subject.
